# EUROPEAN PATENT SPECIFICATION

(11) **EP 2 747 712 B1**
(45) Date of publication and mention of the grant of the patent: **12.10.2016**
(21) Application number: 12755803.9
(22) Date of filing: 03.08.2012
(51) Int. Cl.: A61F 2/30, A61F 2/38, A61F 2/42

(54) **JOINT PROSTHESIS**
GELENKPROTHESE
PROTHÈSE D'ARTICULATION

(30) Priority: 23.08.2011 EP 11006873
(43) Date of publication of application: 02.07.2014
(73) Proprietor: Westfälische Wilhelms-Universität Münster, 48149 Münster (DE)
(72) Inventor: RASCHKE, Michael J., 48145 Münster (DE); LANGER, Martin, 59423 Unna (DE)
(74) Representative: Patent- und Rechtsanwälte Ullrich & Naumann
(86) International application number: PCT/EP2012/065211
(87) International publication number: WO 2013/026676

(56) References cited:
- WO-A1-2010/054400
- GB-A- 2 159 416
- US-A1- 2002 165 614
- US-A1- 2006 142 866
- US-A1- 2006 224 243

## Description

The present invention relates to a joint prosthesis for the articulation of a first bone with respect to a second bone of a human patient, comprising a first bone engaging element being configured to be arranged and supported on the end of said first bone that faces said second bone, and an articular head element having an adjoining portion adapted to cooperate and anatomically articulate with the end of the second bone that faces said first bone. Such a joint prosthesis is known from US 2006/0142866 A1.

Joint prostheses for the articulation of a first bone with respect to a second bone of a human patient are known in prior art in various forms. The present invention predominantly relates to the articulation between long bones. Correspondingly, the disadvantages and problems inherent to respective prior art prostheses are exemplary outlined hereinafter with respect to the elbow as a typical joint in which long bones are involved, or more specifically with respect to the articulation between a radius and a humerus.

The elbow is a complex articulated joint formed between the distal end of the humerus, extending from the shoulder to the elbow, and the proximal ends of the radius and ulna, which lie essentially parallel to each other to form the forearm. The elbow joint consists of three parts, which are the humeroulnar joint (articulatio humeroulnaris), the proximal radioulnar joint (articulatio radioulnaris proximalis), and the humeroradial joint (articulatio humeroradialis), wherein the present invention particularly relates to the latter. The humeroradial joint is the joint between the radial head - which constitutes the proximal end of the radius - and the capitulum of the humerus. It is a limited ball-and-socket joint having significant importance for the stability of the elbow joint.

With regard to the radial head, many different kinds of damages or destruction of the same can be observed. Typically, radial head fractures occur as a result of falls either on the outstretched hand or on the elbow itself. Nowadays, in case of radial head damages or destructions, the standard procedure to normalize anatomic structures and to remediate functional restrictions is the operative excision of the radial head together with an implantation of a prosthesis into the radius in order to replace the radial head.

In prior art various radial head prostheses exist, for instance dual head prostheses (also known as bipolar prostheses), e.g., according to Judet. Typically, such prosthesis includes a stem component configured for being implanted into the intramedullary canal of the proximal radius bone. The stem component is surmounted in proximal direction by a neck portion integral with a ball- or tear-shaped projecting engagement member. Furthermore, this type of prosthesis includes a radial head component comprising a concave face that is designed to anatomically articulate with the convexity of the humerus capitulum to form an atomic joint surface contact area. The radial head component further comprises a cavity for receiving the ball- or tear-shaped projection of the stem component.

This kind of prior art prosthesis for the radial head comes along with several disadvantages.

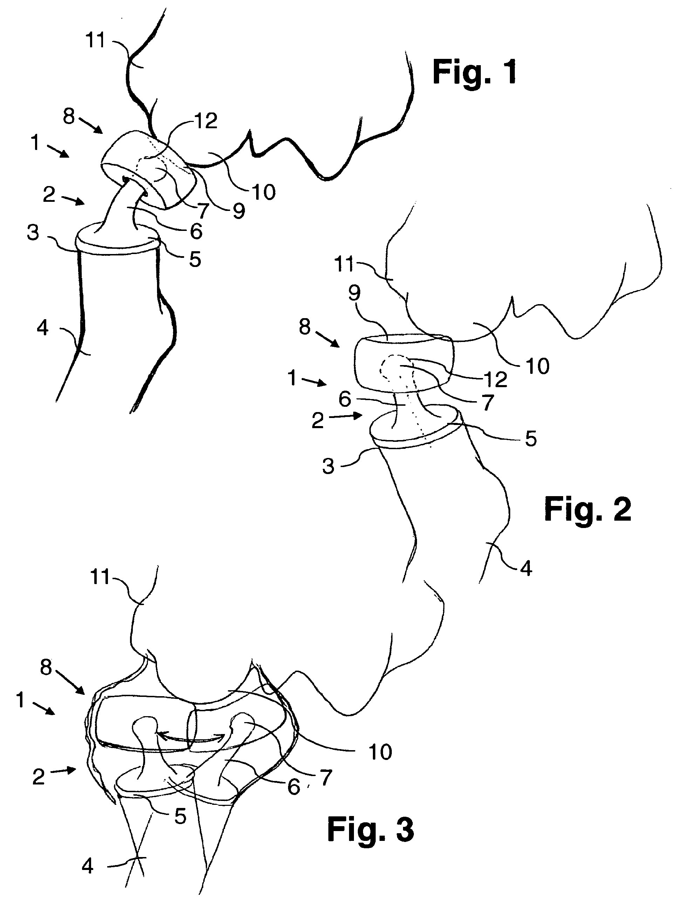

First, as mentioned above and as illustrated in Fig. 1, the concave articular surface on the proximal side of the radial head component anatomically articulates with the convexity of the capitulum of the humerus, while the projection formed at the proximal end of the stem component is engaged with the cavity formed in the radial head component. Due to this arrangement the prosthesis is rather stiff, since deflection between the radius and the humerus of a certain degree result in the inner wall of the cavity of the radial head component abutting against the neck portion of the stem component, thereby prohibiting further movement. In particular when during the operation the stem component has been anchored within the radius bone with a slight misalignment, such that the orientation of the neck portion with respect to the radius is not optimal, it may happen that the distal centre of the radial head component can not entirely compensate such misalignment. This situation is illustrated in Fig. 2. In a worst case scenario the concave articular surface of the radial head component may even become dislocated from the convexity of the capitulum of the humerus.

Secondly, as can be obtained from Fig. 3 the prosthesis replacing the radial head relies on the anular ligament that supports the prosthesis in being positioned in a stable fashion. However, as explained before, constrictions regarding the freedom of movement of the radial head component result in slight movements of the radius bone with respect to the ulna, to which the anular ligament adheres. As a result thereof the anular ligament is exposed to recurrent stretching, illustrated in Fig. 3, so that the ligament structure is very likely to finally wear out, which in turn may cause early relaxation of the prosthesis.

Thirdly, as is best appreciated in Fig. 3, the anular ligament runs from the anterior margin of the radial notch of the ulna around the radial head to the posterior margin of the radial notch. With a prosthesis according to prior art in place, the anular ligament comes to lie in a position which at least partly extends below the distal end of the radial head component, i.e. the anular ligament essentially surrounds the radius at the level of the neck portion of the stem component. As a consequence, when the radial head component performs movements such that it becomes inclined with respect to the stem component or the neck portion thereof, respectively, the anular ligament may be injured through being crushed or clamped in between.

It is therefore an objective of the present invention to improve and further develop a joint prosthesis of the initially described type in such a way that an articulation between the first bone and the second bone is established that restores functional usefulness to the limbs concerned as far as possible without the above mentioned drawbacks.

In accordance with the invention, the aforementioned objective is accomplished by a joint prosthesis comprising the features of claim 1. According to claim 1 such a joint prosthesis is characterized in that said articular head element includes at its end that faces said first bone a projecting engagement member and that said first bone engaging element includes a cavity for receiving said projecting engagement member.

According to the present invention it has first been recognized that the drawbacks of prior art prostheses can be efficiently minimized or even eliminated by inverting the principle known in prior art from dual head prostheses. This means that, compared to prior art prostheses, the allocation of the projecting engagement member and of the cavity, in which the projecting engagement member movably engages, is switched. More specifically, the projecting engagement member is configured to be part of the articular head element, whereas the cavity for receiving the projecting engagement member is configured to be part of the first bone engaging element.

By means of this construction, the center of rotation is shifted more towards the end of the first bone. For instance, in case of applying the principle of the present invention to the radial head, a distalization of the center of rotation is achieved, resulting in that the center of rotation becomes located close to the original anatomic center of rotation. Furthermore, due to the distal shifting of the center of rotation the articular head element is enabled to better compensate movements. As a consequence, ligament structures concerned, e.g. the anular ligament in case of a radial head prosthesis, are widely preserved from getting gradually worn out.

According to a preferred embodiment the first bone engaging element includes a stem component to be anchored within the first bone. The stem component may be made of a resistant material, such as titanium or cobalt chrome. In order to facilitate implantation into the bone tissue, e.g. into the medullary canal of a proximal radius bone, the stem component may be slightly tapered with a decreasing cross section and/or may comprise grooves extending in a longitudinal direction of the stem component. In a specific embodiment the stem component may be constructed in form of an expansible plug including a longitudinally divided expansible shank, wherein the two shank-halves are expandable by way of screw insertion.

Adjacent to the stem component the first bone engaging element may be equipped with a receptacle component. Preferably, the receptacle component is integral with the stem component and is fabricated of the same material. The cavity for receiving the projecting engagement member of the articular head element may be formed within the receptacle component.

In a specific embodiment it may be provided that the receptacle component is formed as a truncated cone body. The truncated cone body may include a lower surface of generally anular shape, the diameter of which is adapted to the diameter of the end portion of the respective first bone. Furthermore, the lower surface of the truncated cone body may extend essentially perpendicular to the stem component, and it may be adapted for abutting against an end surface of the first bone when the stem component is completely implanted into the first bone. However, depending on the specific application area, the engaging element may also be fabricated with the longitudinal axes of the stem component and of the truncated cone body not being aligned with each other, but having a certain angle enclosed therebetween. As will be explained later in more detail, such inclination may be required, for instance, in case the prosthesis is deployed as capitulum replacement between the distal end of a humerus bone and the proximal end of the corresponding radius bone.

In case of the receptacle component being formed as a truncated cone body, the cavity for receiving the projecting engagement member of the articular head element may generally be defined by the lower surface and by the tapered side walls of the truncated cone component. The inner shape of the cavity will be adapted to the form of the projecting engagement member in order to enable smooth sliding motion of the projecting engagement member within the cavity. The opening of the cavity may be formed such that the cavity functions as a kind of snap or catch socket, i.e. the projecting engagement member can be pressed into the cavity with a snug fit.

According to a preferred embodiment the articular head element includes a base component with an at least generally circular outer surface, e.g. from below the adjoining portion at the one end of the articular head element to above the projecting engagement member at the other end of the articular head element. In particular, the base component may essentially be pear-shaped. In such embodiment the involved ligament structure, for instance the anular ligament in case of a radial head prosthesis, would be able to smoothly loop over the base component without any risk of getting injured by way of clamping or crushing.

Depending on the specific application area the adjoining portion of the articular head element adapted to cooperate with the end of the second bone that faces said first bone may be constructed in different ways. For instance, in case of a prosthesis intended to serve as a radial head prosthesis the adjoining portion may be constructed as an essentially concave recess that articulates with the convexity of the humerus capitulum. On the other hand there are applications, for instance distal radioulnar joint prostheses, in which the adjoining portion may be constructed as a spherical head, which has to be partly milled into the radioulnar fusion mass.

With respect to a highly free sliding movement of the projecting engagement member within the cavity of the articular head element, the projecting engagement member may have a form which is essentially ball- or tear-shaped. In particular, due to stability considerations this ball- or tear-shaped projection may be formed integral with the base component of the articular head element.

According to a particularly preferred embodiment the articular head element comprises a telescopic mechanism that allows for intraoperative adjustment of the length of the articular head element. By the provision of such telescopic mechanism it becomes possible to adapt the prosthesis to a patient's specific anatomic conditions, thereby drastically reducing the requirement of follow-up surgical operations.

In a specific embodiment the telescopic mechanism may be realized by providing an articular head element that comprises a first head component including the adjoining portion adapted to cooperate with the end of the second bone that faces the first bone, and a second head component including the projecting engagement member. The first head component and the second head component may then be arranged longitudinally displaceably with respect to each other. In such case, due to stability considerations it proves suitable that the projecting engagement member be formed integral with the second head component of the articular head element.

In order to realize a longitudinally displaceability as mentioned above, the first head component may include a blind hole located concentrically with a longitudinal axis of the articular head element. In addition, the second head component may include an essentially cylindrical body that is arranged within the blind hole of the first head component to be slidably movable along the longitudinal axis of the articular head element. The inner diameter of the blind hole of the first head component and the outer diameter of the essentially cylindrical body of the second head component may be chosen such that the latter is guided within the former in a longitudinal direction without any transverse play.

With respect to a facile blocking of displaceability in order to secure the articular head element in a position after having performed the adjustment, it may be provided that the essentially cylindrical body of the second head component includes an elongated hole bore that is aligned in a rotational direction with a hole provided in the circumferential surface of the first head component for receiving a fastening screw that acts between the first head component and the second head component.

As alternative or additional means, the inner surface of the blind hole and the outer surface of the essentially cylindrical body may include mutually cooperating means for adjusting the relative longitudinal positioning of the first head component with respect to the second head component. For instance, these mutually cooperating means may include a thread, such that the second head component can be screwed into the blind hole of the first head component up to a desired position. Alternatively, a catch mechanism may be provided.

As already mentioned above, in a preferred embodiment the prosthesis will be adapted for the articulation of a radius bone with respect to a humerus bone. To this end the first bone engaging element may be formed as a radius engaging element to be arranged and supported on the proximal end of a radius bone. Moreover, the adjoining portion of the articular head element may be adapted to cooperate with the distal end of a humerus bone or, more specifically, the face of the articular head element may be shaped in form a concave recess for engagement with the capitulum of the humerus to allow for relative sliding motion there between.

In another embodiment the prosthesis is adapted to function as distal radioulnar joint prosthesis, wherein the first bone engaging element is formed as an ulna engaging element to be arranged and supported on the distal end of an ulna bone. In such case, the adjoining portion of the articular head element may be constructed as a spherical head adapted for articulating with the ulnar notch of the corresponding radius bone.

As will be apparent to a person skilled in the pertaining art, the deployment of the prosthesis according to the present invention is not restricted to the two applications mentioned above. In fact, an implantation of the prosthesis according to the present invention can be considered in various constellations, as the case may be with minor adaptions of the prosthesis to the specific anatomic characteristics of the respective joint.

There are several ways how to design and further develop the teaching of the present invention in an advantageous way. To this end, it is to be referred to the patent claims subordinate to patent claim 1, and to the following explanation of preferred examples of embodiments of the invention illustrated by the drawing on the other hand. In connection with the explanation of the preferred examples of embodiments of the invention by the aid of the drawing, generally preferred embodiments and further developments of the teaching will be explained. In the drawings
- Fig. 1: is a schematic view illustrating a radial head prosthesis according to prior art with well adapted positioning,
- Fig. 2: is a schematic view illustrating a radial head prosthesis according to prior art with misaligned positioning,
- Fig. 3: is a schematic view illustrating movements of a radial head prosthesis according to prior art,
- Fig. 4: is a schematic view illustrating a radial head prosthesis according to an embodiment of the present invention,
- Fig. 5: is a schematic view illustrating an articular head element together with an engaging element of a prosthesis according to an embodiment of the present invention,
- Fig. 6: is a schematic view illustrating an ulna head prosthesis according to an embodiment of the present invention,
- Fig. 7: is a schematic view illustrating a prosthesis according to an embodiment of the present invention that functions as humerus capitulum replacement,
- Fig. 8: is a schematic view illustrating an alternative embodiment of an articular head element of a prosthesis in accordance with the present invention,
- Figs. 9a, b: are schematic cross-sections illustrating a telescopic mechanism of an articular head element according to an embodiment of the present invention,
- Figs. 10a, b: are schematic cross-sections illustrating a telescopic mechanism of an articular head element according to another embodiment of the present invention, and
- Figs. 11a, b: are schematic views illustrating an embodiment of an engaging element with expansible shaft in accordance with the present invention.

Fig. 1 schematically illustrates a prior art dual head (or bipolar) prosthesis 1 according to Judet intended for replacing the radial head of a human patient. The illustrated prosthesis 1 includes a stem component 2 that is being implanted into the intramedullary canal of the proximal end 3 of a radius bone 4. The stem component 2 is surmounted in proximal direction by a neck portion 5 integral with a projecting engagement member 6 having a ball- or tear-shaped proximal end 7. Furthermore, the prosthesis 1 includes a radial head component 8 comprising a concave face 9 that is designed to anatomically articulate with the convexity of the capitulum 10 of a humerus bone 11 to form an anatomic joint surface contact area. The radial head component 8 further comprises a cavity 12 for receiving the ball- or tear-shaped proximal end 7 of projecting engagement member 6 of the stem component 2.

Fig. 1 shows the prosthesis 1 in an excellent implantation position, in which a perfect articulation between the convexity of the humerus capitulum 10 and the concavity of the radial head component 8 is achieved. In contrast thereto, Fig. 2 illustrates an implantation situation of the same prosthesis 1 with a slightly inappropriate alignment of the stem component 2 within the radius 4. As a consequence of this misalignment, the anatomic joint surface contact area formed between the humerus capitulum 10 and the concave face 9 of the radial head component 8 is drastically reduced. In particular, the distal center of the radial head component 8 is not capable any more to compensate all relative motions between the radius 4 and the humerus 11. Consequently, in certain situations the concave articular surface 9 of the radial head component 8 may even become dislocated or released from the convexity of the capitulum 10 of the humerus 11.

Fig. 3 demonstrates a further drawback of the depicted prior art dual head prosthesis 1. In addition to the illustrations of Figs. 1 and 2, the ulna bone 13 and the corresponding ligament structure is also depicted Fig. 3. In this context the so called anular ligament 14 is of importance, which attaches on the front and on the back side of the radial notch of the ulna 13, encompassing the head of the radius 4 without attaching to it and holding it against the ulna 13.

As can be obtained from Fig. 3, the anular ligament 14 encircles the radius 4 substantially at a position distal to the distal end of the radial head component 8 and proximal to the neck portion 5 of the stem component 2. Consequently, when the radial head component 8 comes into an inclined position with respect to the stem component 2, the anular ligament 13 may be injured or damaged by possibly being clamped or crushed between the radial head component 8 and the neck portion 5 of the stem component 2.

Furthermore, misalignments as discussed in connection with Fig. 2 might cause slight motions of the radius 4 with respect to the ulna 13, resulting in the risk of the anular ligament 14 getting prematurely worn out.

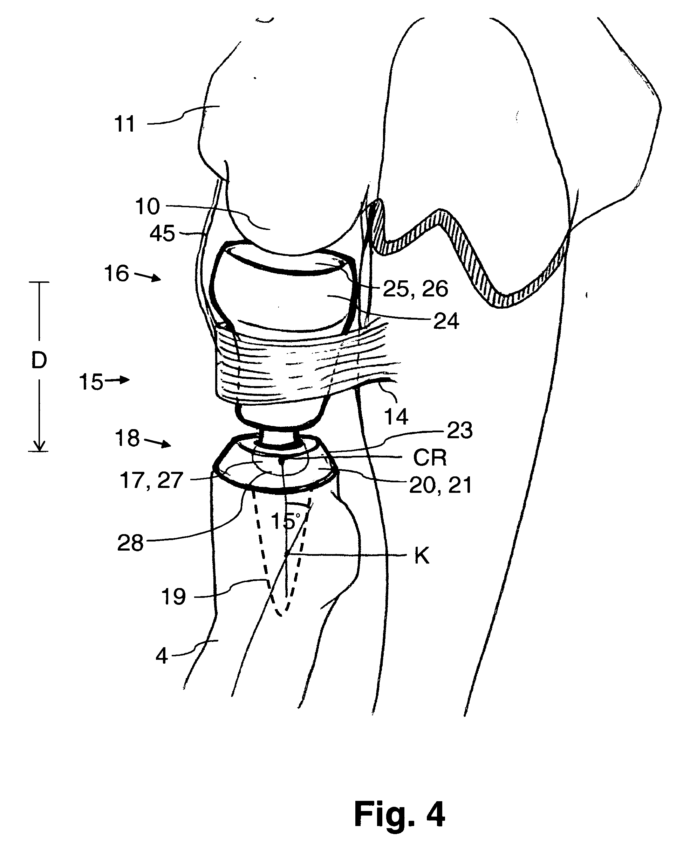

Fig. 4 schematically illustrates a first embodiment of a prosthesis according to the present invention. Specifically, the embodiment relates to a radial head prosthesis 15 for the articulation of a radius bone 4 with respect to a humerus bone 10.

In contrast to the prior art prostheses described in connection with Figs. 1-3, the prosthesis 15 according to the invention is inverted. More specifically and as will be explained in more detail hereinafter, the prosthesis 15 comprises an articular head element 16 including at its distal end a projecting engagement member 17, and an engaging element 18 for the radius 4 including a cavity 28 for receiving the projecting engagement member 17. Hence, the center of rotation (CR) is shifted more towards the 15° kink (K) close to the proximal end of the radius 4, such that a distalization (D) of the rotation is achieved. As a result, a significant improvement with respect to the protection of both the anular ligament 14 and the capsule 45 is obtained.

The radius engaging element 18 includes a stem component 19 which is implanted during an operation into the intramedullary canal of a patient's proximal radius bone 4. As will be apparent to a person skilled in the art, the stem component 19 may comprise various means to facilitate its anchoring in the radius 4. For instance, the stem component 19 may be equipped with grooves extending in a longitudinal direction of the stem component 19, and/or it may be slightly tapered with a decreasing cross section. With respect to good preservability and long-lasting durability the radius engaging element 18, and in particular the stem component 19, may be fabricated of a resistant material, such as titanium or chrome cobalt.

Adjacent to the stem component 19 in proximal direction the radius engaging element 18 includes a receptacle component 20. To ensure highest stability, the receptacle component 20 is formed integral with and of the same material as the stem component 19. In the embodiment of Fig. 4, the receptacle component 20 is fabricated as an essentially truncated cone body 21. However, any other form that is suitable for receiving the projecting engagement member 17 of articular head element 16 may be realized.

The truncated cone body 21 of the receptacle component 20 includes a lower surface 22 of generally angular shape that extends essentially perpendicular to the stem component 19. The lower surface 22 is adapted for abutting against the proximal end surface of the radius bone 4. Preferably, the radius of the lower surface 22 may be formed to correspond with the radius of the proximal end surface of the radius bone 4. Hence, it proves to be advantageous to provide engaging elements 18 with different dimensions, in particular with different radii of lower surface 22, from which the surgeon may choose during surgery according to the patient's specific needs.

The truncated cone body 21 further includes tapered side walls 23 such that the outer diameter of the truncated cone body 21 at the proximal end thereof is reduced compared to the outer diameter at the distal end thereof. In particular, as can be obtained from Fig. 4, the outer diameter of truncated cone body 21 at the proximal end thereof may be configured to correspond with the outer diameter of the distal end of the articular head element 16. Through the lower surface 22 together with the tapered side walls 23 a cavity 28 for receiving the projecting engagement member 17 is established inside the truncated code body 21.

In the embodiment of Fig. 4, the articular head element 16 includes a base component 24 having an essentially pear-shaped form tapering in distal direction. Between its distal end and its proximal end, the base component 24 is surrounded and stabilized in its position by the anular ligament 14. Due to the continuous formation of the outer side walls of the base component 24, a smooth contact surface is established for the anular ligament 14, such that clamping or crushing of the anular ligament 14 is almost impossible. Similar to the case of the engaging elements 18, in standard practice the base components 24 could be offered in a number of different sizes, e.g. small, medium and large, from which the surgeon may choose during surgery according to the patient's specific needs.

The proximal end of the base component 24 is formed by an adjoining portion 25 that is shaped in form a concave recess 26 for engagement with the capitulum 10 of the humerus 11 to allow for relative sliding motion there between. As already mentioned above, at its distal end the articular head element 16 includes a projecting engagement member 17, which is formed integral with the base component 24 of the articular head element 16. Both the base component 24 and the projecting engagement member 17 may be fabricated of a persistent material, like for instance titanium or chrome cobalt.

As can be seen from Fig. 4, the projecting engagement member 17 is configured to include an essentially ball- or tear-shaped projection 27. The specific form of the ball- or tear-shaped projection 27 is configured to correspond with the inner form of the cavity 28 to allow for relative sliding motion there between.

By the inversion of the principle known from prior art dual head prostheses, i.e. by assigning the male engagement part (or more specifically the projecting engagement member 17) to the articular head element 16 and by assigning the female engagement part (or more specifically the receptacle component 20) to the radius engaging element 18, the present invention achieves a situation in which the rotation center of the prosthesis 15 is shifted in a distal direction. As a consequence, the prosthesis 15 according to the present invention has a rotation center being located close to the original anatomic rotation center.

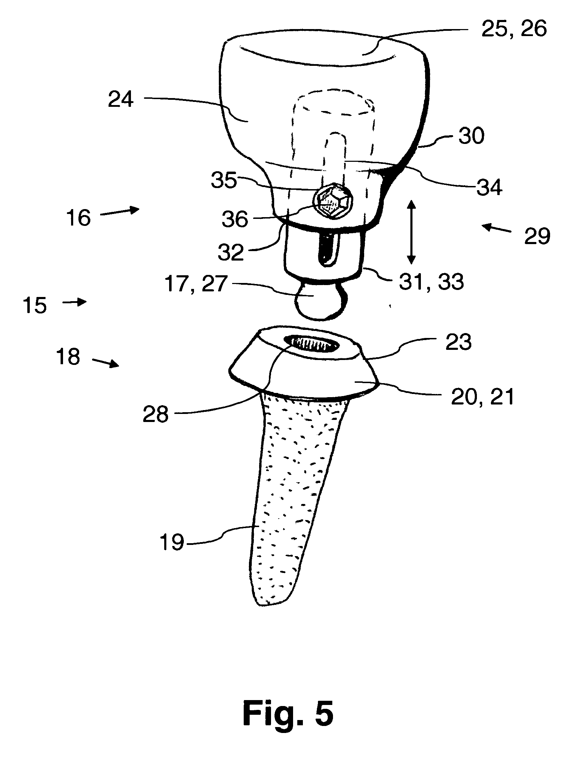

Fig. 5 schematically illustrates an articular head element 16 together with an engaging element 18 of a radial head prosthesis 15 according to a second embodiment of the present invention. The same reference numerals denote the same components or elements as in connection with Fig. 4. The articular head element 16 includes a telescopic mechanism 29 for intraoperative adjustment of the length of the articular head element 16, as indicated by the double arrow.

In the embodiment of Fig. 5, the articular head element 16 is composed of two parts: a first head component 30 including the preferably concave face 25 adapted to cooperate with the capitulum 10 of the humerus 11, and a second head component 31 including the projecting engagement member 17. The first head component 30 and the second head component 31 are arranged longitudinally displaceably with respect to each other.

More specifically, the first head component 30 includes a blind hole 32 that is located concentrically with a longitudinal axis of the articular head element 16 and that extends from the distal end of the first head component 30 to below the concave face 25 at the proximal end of the first head component 30. On the other hand, the second head component 31 including the projecting engagement member 17 comprises an essentially cylindrical body 33 that is arranged within the blind hole 32 to be slidable along the longitudinal axis of the articular head element 16. The inner diameter of the blind hole 32 of the first head component 30 and the outer diameter of the essentially cylindrical body 33 of the second head component 31 are adapted such that the latter is guided within the former in a longitudinal direction without any transverse play.

After the surgeon during surgery has adjusted the optimal length of the articular head element 16, a blocking mechanism can be actuated in order to fix and enduringly secure the relative position of the first head component 30 and the second head component 31 with respect to each other. To this end the essentially cylindrical body 33 of the second head component 31 includes an elongated hole bore 34 that can be aligned in a rotational direction with a hole 35 provided in the circumferential surface of the first head component 30 for receiving a fastening screw 36.

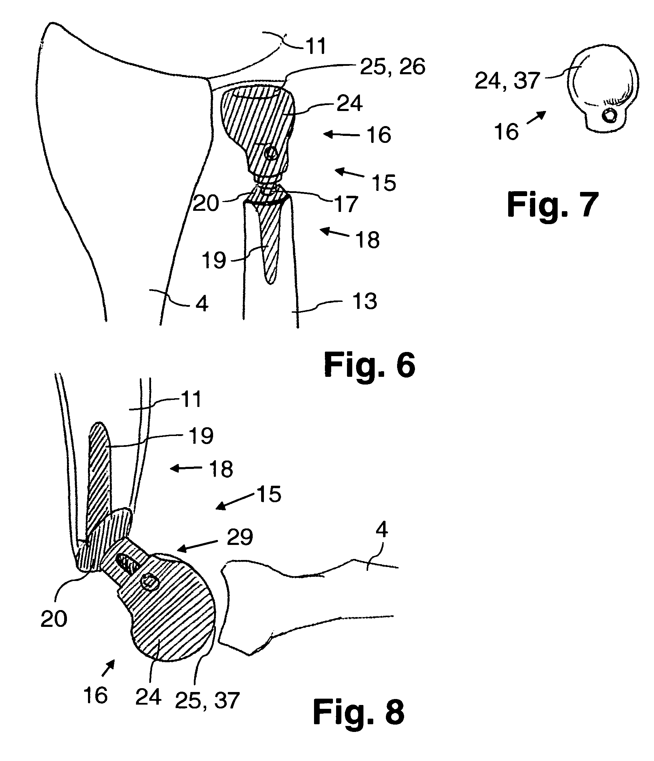

Fig. 6 is a schematic view illustrating another embodiment of a prosthesis according to the present invention, which is employed as an ulna head prosthesis or, more specifically, as a distal radioulnar joint prosthesis 15. The same reference numerals denote the same components or elements as in connection with Figs. 4 and 5. Construction and design of the prosthesis 15 are rather similar to the radial head prosthesis of Fig. 4. Again, the principle known from prior art dual head prostheses is inverted, meaning that the male engagement part, which is projecting engagement member 17, is assigned to the articular head element 16, and the female engagement part, which is receptacle component 20, is assigned to the ulnar engaging element 18.

The ulnar engaging element 18 includes a stem component 19 which is implanted, for instance after a Sauvé-Kapandji operation into the distal end of a stump of a patient's ulna bone 13. Adjacent to the stem component 19 in distal direction, the ulnar engaging element 18 includes a receptacle component 20. To ensure highest stability, the receptacle component 20 is formed integral with and of the same material as the stem component 19.

On the other hand, the articular head element 16 includes a base component 24 having an essentially pear-shaped form. The distal end of the base component 24 is formed by an adjoining portion 25 that is shaped in form a concave recess 26 for engagement with the ulnar notch of the corresponding radius bone 4 to allow for relative sliding motion there between.

Alternatively, as is shown in Fig. 7, the articular head element 16 may include a base component 24 that, instead of having an essentially pear-shaped form, has the form of a spherical head 37. This kind of prosthesis proves to be particularly advantageous after failed Sauvé-Kapandji procedures for treatment of a painful complication in form of radioulnar convergence. Basically, the prosthesis is implanted like it is shown in Fig. 6, however, the spherical head 37 of the base component 24 will be milled into the ulnar notch of the corresponding radius bone 4. Details of this kind of treatment are described in Diego L. Fernandez et al.: "Treatment of Failed Sauvé-Kapandji Procedures with a Spherical Ulnar Head Prosthesis", in CLINICAL ORTHOPAEDICS AND RELATED RESEARCH, No. 45, pp. 100-107. In addition to the positive results achieved by the treatment described therein, by respective placement of a prosthesis according to the present invention one achieves an additional advantage as follows:

The spherical head of a prosthesis according to Diego Fernandez has to be mounted into a fixed system. To this end, prior to inserting the prosthesis the radius bone has to be cut through and opened by means of a spreader clamp as illustrated in Fig. 3 of the above mentioned document. After insertion of the prosthesis the radius has to be closed again and to be screwed together with a plate osteosynthesis which, altogether, is a rather complex and laborious procedure. In contrast, a prosthesis in accordance with an embodiment of the present invention including a telescopic mechanism can be inserted with the telescopic mechanism being in a retracted (i.e. not extended) state. After insertion, the telescopic mechanism can be extended in order to adjust and fix the length of the prosthesis in situ. As a consequence, there is no need of cutting through and/or opening the radius.

Fig. 8 is a schematic view illustrating still another embodiment of a prosthesis according to the present invention, which is employed as a humerus capitulum replacement. Again, same reference numerals denote the same components or elements as in connection with the previous Figs. 4-7.

In this embodiment the engaging element is a humerus engaging element 18, i.e. the stem component 19 of the engaging element 18 is implanted into the distal end of a patient's humerus bone 11. Adjacent to the stem component 19 in distal direction, the humerus engaging element 18 includes a receptacle component 20. Like in the embodiments described above, to ensure highest stability, the receptacle component 20 is formed integral with and of the same material as the stem component 19.

On the other hand, the articular head element 16 includes a base component 24 having an adjoining portion 25 which is adapted to cooperate with the proximal end of the radius bone 4. In order to achieve a smooth articulation, the base component 24 is constructed as a spherical head 37, thereby providing at the distal end of the base component 24 an adjoining portion 25 with a spherical face that articulates with the capitulum of the radius bone 4. For the purpose of exact intraoperative adjustment of the length of the prosthesis 15, the articular head element 16 comprises a telescopic mechanism 29, which in the embodiment shown is the same as the one described in connection with Fig. 4.

In contrast to the previous embodiments, the stem component 19 of the humerus engaging element 18 is not aligned with the central axis of the receptacle component 20. In fact, in order to adapt the prosthesis 15 to the specific anatomic characteristics of the joint between the capitulum of the humerus 11 and the proximal end of the radius 4, the stem component 19, as shown in a slightly exaggerated illustration in Fig. 8, is inclined by an angle of approximately 25° with respect the central axis of the receptacle component 20.

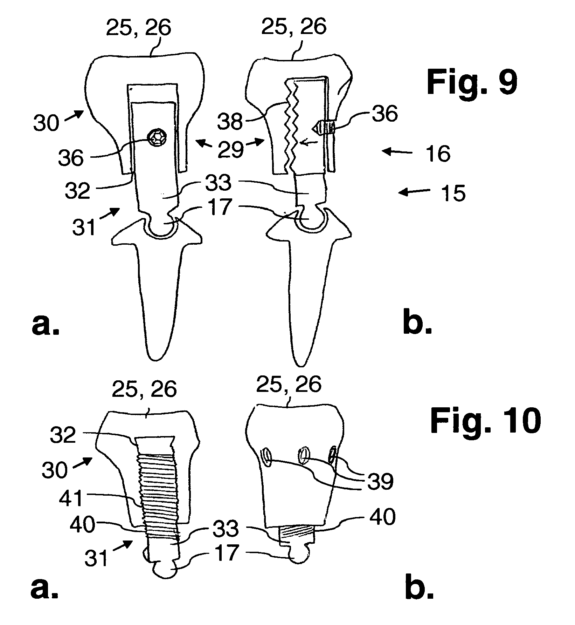

Figs. 9a, b and 10a, b illustrate further embodiments of a telescopic mechanism for controlling interoperatively the precise fitting of the prosthesis by adjusting the length of the articular head element 16 of the prosthesis 15. In both embodiments, the articular head element 16 of the prosthesis 15 is composed of a first head component 30 and a second head component 31. The first head component 30 includes the adjoining portion 25 adapted to cooperate with the end of the second bone that faces the first bone. In both embodiments shown the adjoining portion 25 is a concave face 26. The second head component 31 includes an essentially cylindrical body 33 with the projecting engagement member 17 being integrally formed at one end thereof. The body 33 is movably arranged within a blind hole 32 provided inside the first head component 30 concentrically with a longitudinal axis of the same.

With reference to the embodiment of Figs. 9a, b, the telescopic mechanism 29 includes a catch mechanism 38 for adjusting the relative longitudinal positioning of the first head component 30 with respect to the second head component 31. More specifically, serrated structures are provided in a certain section of the peripheral surfaces of both the body 33 and the inner surface of the blind hole 32. These serrated structures may be sawtooth in cross-section, as can be obtained from the sectional view of Fig. 9b. A tapped hole 39 is provided within the side face of the first head component 30 at a position essentially opposite of the serrated structure. By means of screwing a fastening screw 36, for instance a grub screw, into the tapped hole 39, a contact pressure (as indicated by the arrow) will be exerted on the serrated structures, which in turn results in a secure and stable fixation between the first and the second head component 30, 31.

Due to the spatial extension of the saw teeth of the serrated structure, the telescopic mechanism 29 described above does not provide the possibility of an infinitely variable adjustment of the length of the prosthesis. If this is desired, the embodiment of a telescopic mechanism 29 shown in Figs. 10a, b appears to be more suitable. In this case, the essentially cylindrical body 33 of the second head component 33 comprises an external thread 40 acting together with a corresponding internal thread 41 that is provided at the inner surface of the blind hole 32 of the second head component 31. By screwing in the cylindrical body 33 of the second head component 31 into the blind hole 32 of the first head component 30, any desired length of the prosthesis can be realized in a continuous fashion.

Like in the embodiment of Figs. 9a, b, the fixation between the first and the second head component 30, 31 can be achieved by means of a fastening screw 36, which is screwed into a tapped hole 39 provided within the side face of the first head component 30, once the desired length of the prosthesis has been set. As illustrated in Fig. 10b, in order to enhance the reliability and stability of the fixation, a plurality of tapped holes 39 and fastening screws 36 may be employed along the circumferential surface of the first head component 31.

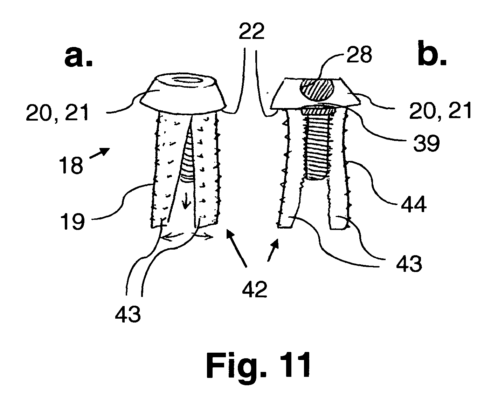

Figs. 11a, b are schematic views illustrating an embodiment of an engaging element 18 with expansible shaft in accordance with the present invention. Like the engaging element employed in the prosthesis described in connection with Fig. 4, the engaging element 18 includes a stem component 19, which is to be anchored within the first bone, and a receptacle component 20 being formed integral with and of the same material as the stem component 19. The receptacle component 20 is fabricated as an essentially truncated cone body 21 inside of which a cavity 28 for receiving the projecting engagement member 17 of the corresponding articular head element (not shown) is established.

The stem component 19 of the engaging element 18 is constructed in form of an expansible plug 42 including a longitudinally divided expansible shank, wherein the two shank-halves 43 are expandable (as indicated by the opposing arrows) by way of screw insertion. More specifically, a tapped hole 39 is provided within the lower surface 22 of the truncated cone body 21, such that screwing in of a screw can be accomplished via the cavity 28. In addition, a plurality of spikes 44 are provided at the outer surfaces of the two shank halves 43 in order to facilitate secure and stable anchoring of the stem component 19 within the bone.

Many modifications and other embodiments of the invention set forth herein will come to the mind of the one skilled in the art to which the invention pertains, having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### Reference List

- 1: prosthesis
- 2: stem component
- 3: proximal end (radius bone)
- 4: radius bone
- 5: neck portion
- 6: projecting engagement member
- 7: ball- or tear shaped proximal end of 6
- 8: radial head component
- 9: concave face
- 10: humerus capitulum
- 11: humerus bone
- 12: cavity
- 13: ulna bone
- 14: anular ligament
- 15: prosthesis
- 16: articular head element
- 17: projecting engagement member
- 18: engaging element
- 19: stem component
- 20: receptacle component
- 21: truncated cone body
- 22: lower surface of 21
- 23: tapered side walls of 21
- 24: base component
- 25: adjoining portion
- 26: concave recess
- 27: ball- or tear-shaped projection
- 28: cavity
- 29: telescopic mechanism
- 30: first head component
- 31: second head component
- 32: blind hole
- 33: cylindrical body
- 34: elongated hole bore
- 35: hole
- 36: fastening screw
- 37: spherical head
- 38: catch mechanism
- 39: tapped hole
- 40: external thread
- 41: internal thread
- 42: expansible plug
- 43: shank-halves
- 44: spikes
- 45: capsule

## Claims

1. Joint prosthesis for the articulation of a first bone with respect to a second bone of a human patient, comprising a first bone engaging element (18) being configured to be arranged and supported on the end of said first bone that faces said second bone, and an articular head element (16) having an adjoining portion (25) adapted to cooperate and anatomically articulate with the end of the second bone that faces said first bone,
**characterized in that** said articular head element (16) includes at its end that faces said first bone a projecting engagement member (17) and that said first bone engaging element (18) includes a cavity (28) for receiving said projecting engagement member (17).

2. Prosthesis according to claim 1, wherein said first bone engaging element (18) includes a stem component (19) to be anchored within said first bone.

3. Prosthesis according to claim 2, wherein said first bone engaging element (18) includes a receptacle component (20) adjacent to said stem component (19).

4. Prosthesis according to claim 3, wherein said cavity (28) for receiving said projecting engagement member (17) of said articular head element (16) is formed within said receptacle component (20).

5. Prosthesis according to claim 3 or 4, wherein said receptacle component (20) is formed as an essentially truncated cone body (21), preferably including a lower surface (22) of generally annular shape that extends essentially perpendicular to said stem component (19) and that is adapted for abutting against an end surface of said first bone.

6. Prosthesis according to any of claims 1 to 5, wherein said articular head element (16) includes a base component (24) having an at least generally circular outer surface, said base component (24) preferably being essentially pear-shaped, and/or
wherein said adjoining portion (25) of said articular head element (16) adapted to cooperate and/or anatomically articulate with the end of the second bone that faces said first bone is constructed as an essentially concave recess (26) or as a spherical head (37).

7. Prosthesis according to any of claims 1 to 6, wherein said projecting engagement member (17) includes an essentially ball- or tear-shaped projection (27), preferably formed integral with said base component (24).

8. Prosthesis according to any of claims 1 to 7, wherein said articular head element (16) comprises a telescopic mechanism (29) for intraoperative adjustment of the length of said articular head element (16).

9. Prosthesis according to any of claims 1 to 8, wherein said articular head element (16) comprises
a first head component (30) including said adjoining portion (25) adapted to cooperate and/or anatomically articulate with the end of the second bone that faces said first bone, and
a second head component (31) including said projecting engagement member (17),
wherein said first head component (30) and said second head component (31) are arranged longitudinally displaceably with respect to each other.

10. Prosthesis according to claim 9, wherein said first head component (30) includes a blind hole (32) located concentrically with a longitudinal axis of said articular head element (16), and wherein said second head component (31) includes an essentially cylindrical body (33) that is arranged within said blind hole (32) to be movable along the longitudinal axis of said articular head element (16).

11. Prosthesis according to claim 10, wherein the inner surface of said blind hole (32) and the outer surface of said essentially cylindrical body (33) include mutually cooperating means for adjusting the relative longitudinal positioning of said first head component (30) with respect to said second head component (31), in particular a thread (40, 41) or a catch mechanism (38).

12. Prosthesis according to claim 10 or 11, wherein said essentially cylindrical body (33) includes an elongated hole bore (34) that is aligned in a rotational direction with a hole (35) provided in the circumferential surface of said first head component (30) for receiving a fastening screw (36).

13. Prosthesis according to any of claims 1 to 12, adapted for the articulation of a radius bone (4) with respect to a humerus bone (11),
wherein said first bone engaging element (18) is formed as a radius engaging element to be arranged and supported on the proximal end of a radius bone (4), and
wherein said adjoining portion (25) of said articular head element (16) is constructed as an essentially concave recess (26) adapted to cooperate with the distal end of the humerus bone (11).

14. Prosthesis according to any of claims 1 to 13, adapted for the articulation of a radius bone (4) with respect to a humerus bone (11),
wherein said first bone engaging element (18) is formed as a radius engaging element to be arranged and supported on the proximal end of a radius bone (4), and
wherein said adjoining portion (25) of said articular head element (16) is constructed as an essentially concave recess (26) adapted to anatomically articulate with the distal end of the humerus bone (11).

15. Prosthesis according to any of claims 1 to 12, adapted to function as distal radioulnar joint prosthesis,
wherein said first bone engaging element (18) is formed as an ulna engaging element to be arranged and supported on the distal end of an ulna bone (13), and
wherein said adjoining portion (25) of said articular head element (16) is constructed as a spherical head (37) adapted for articulating with the ulnar notch of the corresponding radius bone (4).

## Patentansprüche

1. Gelenkprothese für die Artikulation eines ersten Knochens in Bezug auf einen zweiten Knochen eines menschlichen Patienten, mit einem in den ersten Knochen eingreifenden Element (18), das ausgebildet ist, an dem dem zweiten Knochen zugewandten Ende des ersten Knochens angeordnet und unterstützt zu werden, sowie mit einem Gelenkkopfelement (16) mit einem Anlageabschnitt (25), der angepasst ist, mit dem dem ersten Knochen zugewandten Ende des zweiten Knochens zu kooperieren und anatomisch zu artikulieren,
**dadurch gekennzeichnet, dass** das Gelenkkopfelement (16) an seinem dem ersten Knochen zugewandten Ende ein hervorstehendes Eingriffselement (17) aufweist und dass das in den ersten Knochen eingreifende Element (18) einen Hohlraum (28) zum Aufnehmen des hervorstehenden Eingriffselements (17) aufweist.

2. Prothese nach Anspruch 1, wobei das in den ersten Knochen eingreifende Element (18) ein in dem ersten Knochen verankerbares Schaftteil (19) umfasst.

3. Prothese nach Anspruch 2, wobei das in den ersten Knochen eingreifende Element (18) ein an das Schaftteil (19) angrenzendes Aufnahmeteil (20) umfasst.

4. Prothese nach Anspruch 3, wobei der Hohlraum (28) zum Aufnehmen des hervorstehenden Eingriffselements (17) des Gelenkkopfelements (16) innerhalb des Aufnahmeteils (20) ausgebildet ist.

5. Prothese nach Anspruch 3 oder 4, wobei das Aufnahmeteil (20) als ein im Wesentlichen kegelstumpfförmiger Körper (21) ausgebildet ist, vorzugsweise mit einer im Wesentlichen ringförmigen unteren Fläche (22), die sich im Wesentlichen rechtwinklig zu dem Schaftteil (19) erstreckt und die angepasst ist, an einer Stirnfläche des ersten Knochens anzuliegen.

6. Prothese nach einem der Ansprüche 1 bis 5, wobei das Gelenkkopfelement (16) eine Basiskomponente (24) mit einer zumindest im Wesentlichen kreisförmigen äußeren Oberfläche umfasst, wobei die Basiskomponente (24) vorzugsweise im Wesentlichen birnenförmig ausgestaltet ist, und/oder
wobei der Anlageabschnitt (25) des Gelenkkopfelements (16), der angepasst ist, mit dem dem ersten Knochen zugewandten Ende des zweiten Knochens zusammenzuwirken und/oder anatomisch zu artikulieren, als eine im Wesentlichen konkave Ausnehmung (26) oder als Kugelkopf (37) ausgebildet ist.

7. Prothese nach einem der Ansprüche 1 bis 6, wobei das hervorstehende Eingriffselement (17) einen im Wesentlichen kugel- oder tropfenförmigen Vorsprung (27) aufweist, welcher vorzugsweise integral mit der Basiskomponente (24) ausgebildet ist.

8. Prothese nach einem der Ansprüche 1 bis 7, wobei das Gelenkkopfelement (16) einen Teleskopmechanismus (29) für die intraoperative Anpassung der Länge des Gelenkkopfelements (16) aufweist.

9. Prothese nach einem der Ansprüche 1 bis 8, wobei das Gelenkkopfelement (16)
eine erste Kopfkomponente (30), die den Anlageabschnitt (25) aufweist, der angepasst ist mit dem dem ersten Knochen zugewandten Ende des zweiten Knochens zusammenzuwirken und/oder anatomisch zu artikulieren, und
eine zweite Kopfkomponente (31) umfasst, die das hervorstehende Eingriffselement (17) aufweist,
wobei die erste Kopfkomponente (30) und die zweite Kopfkomponente (31) zueinander longitudinal verschiebbar angeordnet sind.

10. Prothese nach Anspruch 9, wobei die erste Kopfkomponente (30) ein konzentrisch mit einer Längsachse des Gelenkkopfelements (16) angeordnetes Sackloch (32) aufweist, und wobei die zweite Kopfkomponente (31) einen im Wesentlichen zylindrischen Körper (33) aufweist, der entlang der Längsachse des Gelenkkopfelements (16) bewegbar innerhalb des Sacklochs (32) angeordnet ist.

11. Prothese nach Anspruch 10, wobei die Innenfläche des Sacklochs (32) und die Außenfläche des im Wesentlichen zylindrischen Körpers (33) wechselseitig zusammenwirkende Mittel zur Einstellung der relativen Längspositionierung der ersten Kopfkomponente (30) in Bezug auf die zweite Kopfkomponente (31), insbesondere ein Gewinde (40, 41) oder einen Rastmechanismus (38), aufweisen.

12. Prothese nach Anspruch 10 oder 11, wobei der im Wesentlichen zylindrische Körper (33) eine Langlochbohrung (34) aufweist, die in einer Drehrichtung mit einem in der Umfangsfläche der ersten Kopfkomponente (30) bereitgestellten Loch (35) zur Aufnahme einer Befestigungsschraube (36) ausgerichtet ist.

13. Prothese nach einem der Ansprüche 1 bis 12, die für die Artikulation eines Speichenknochens (4) in Bezug auf einen Oberarmknochen (11) angepasst ist,
wobei das in den ersten Knochen eingreifende Element (18) als ein in die Speiche eingreifendes Element ausgebildet ist, um an dem proximalen Ende eines Speichenknochens (4) angeordnet und gestützt zu werden, und
wobei der Anlageabschnitt (25) des Gelenkkopfelements (16) als eine im Wesentlichen konkave Ausnehmung (26) ausgebildet ist, die angepasst ist, um mit dem distalen Ende des Oberarmknochens (11) zusammenzuwirken.

14. Prothese nach einem der Ansprüche 1 bis 13, die für die Artikulation eines Speichenknochens (4) in Bezug auf einen Oberarmknochen (11) angepasst ist,
wobei das in den ersten Knochen eingreifende Element (18) als ein in die Speiche eingreifendes Element ausgebildet ist, um an dem proximalen Ende eines Speichenknochens (4) angeordnet und gestützt zu werden, und
wobei der Anlageabschnitt (25) des Gelenkkopfelements (16) als eine im Wesentlichen konkave Ausnehmung (26) ausgebildet ist, die angepasst ist, um mit dem distalen Ende des Oberarmknochens (11) anatomisch zu artikulieren.

15. Prothese nach einem der Ansprüche 1 bis 12, die angepasst ist, um als distale Radioulnar-Gelenkprothese zu fungieren,
wobei das in den ersten Knochen eingreifende Element (18) als ein in die Elle eingreifendes Element ausgebildet ist, um an dem distalen Ende eines Ellenknochens (13) angeordnet und gestützt zu werden, und
wobei der Anlageabschnitt (25) des Gelenkkopfelements (16) als ein Kugelkopf (37) ausgebildet ist, der zur Artikulation mit der Ellen-Einkerbung des entsprechenden Speichenknochens (4) angepasst ist.

## Revendications

1. Prothèse d'articulation pour l'articulation d'un premier os par rapport à un second os d'un patient humain, comprenant un élément de mise en prise avec le premier os (18) configuré pour être agencé et supporté sur l'extrémité dudit premier os qui fait face audit second os, et un élément de tête articulaire (16) ayant une partie attenante (25) adaptée pour coopérer et s'articuler de manière anatomique avec l'extrémité du second os qui fait face audit premier os,
**caractérisée en ce que** ledit élément de tête articulaire (16) inclut au niveau de son extrémité qui fait face audit premier os un élément de mise en prise en saillie (17) et **en ce que** ledit élément de mise en prise avec le premier os (18) inclut une cavité (28) pour recevoir ledit élément de mise en prise en saillie (17).

2. Prothèse selon la revendication 1, dans laquelle ledit élément de mise en prise avec le premier os (18) inclut un composant formant tige (19) devant être ancré à l'intérieur dudit premier os.

3. Prothèse selon la revendication 2, dans laquelle ledit élément de mise en prise avec le premier os (18) inclut un composant formant réceptacle (20) adjacent audit composant formant tige (19).

4. Prothèse selon la revendication 3, dans laquelle ladite cavité (28) pour recevoir ledit élément de mise en prise en saillie (17) dudit élément de tête articulaire (16) est formée à l'intérieur dudit composant formant réceptacle (20).

5. Prothèse selon la revendication 3 ou 4, dans laquelle ledit composant formant réceptacle (20) est sous la forme d'un corps conique essentiellement tronqué (21), incluant de préférence une surface inférieure (22) de forme généralement annulaire qui s'étend essentiellement perpendiculairement audit composant formant tige (19) et qui est adaptée pour venir en butée contre une surface d'extrémité dudit premier os.

6. Prothèse selon l'une quelconque des revendications 1 à 5, dans laquelle ledit élément de tête articulaire (16) inclut un composant formant base (24) ayant une surface externe au moins généralement circulaire, ledit composant formant (24) étant de préférence essentiellement en forme de poire, et/ou
dans laquelle ladite partie attenante (25) dudit élément de tête articulaire (16) adaptée pour coopérer et/ou s'articuler anatomiquement avec l'extrémité du second os qui fait face audit premier os est construite comme un évidement essentiellement concave (26) ou comme une tête sphérique (37).

7. Prothèse selon l'une quelconque des revendications 1 à 6, dans laquelle ledit élément de mise en prise en saillie (17) inclut une saillie essentiellement en forme de bille ou de larme (27), de préférence formée d'un seul tenant avec ledit composant de base (24).

8. Prothèse selon l'une quelconque des revendications 1 à 7, dans laquelle ledit élément de tête articulaire (16) comprend un mécanisme télescopique (29) pour ajustement peropératoire de la longueur dudit élément de tête articulaire (16).

9. Prothèse selon l'une quelconque des revendications 1 à 8, dans laquelle ledit élément de tête articulaire (16) comprend
un premier composant formant tête (30) incluant ladite partie attenante (25) adaptée pour coopérer et/ou s'articuler de manière anatomique avec l'extrémité du second os qui fait face audit premier os, et
un second composant de tête (31) incluant ledit élément de mise en prise en saillie (17),
dans lequel ledit premier composant de tête (30) et ledit second composant de tête (31) sont agencés longitudinalement de manière déplaçable l'un par rapport à l'autre.

10. Prothèse selon la revendication 9, dans laquelle ledit premier composant de tête (30) inclut un trou borgne (32) placé de manière concentrique avec un axe longitudinal dudit élément de tête articulaire (16), et dans lequel ledit second composant de tête (31) inclut un corps essentiellement cylindrique (33) qui est agencé à l'intérieur dudit trou borgne (32) devant être mobile le long de l'axe longitudinal dudit élément de tête articulaire (16).

11. Prothèse selon la revendication 10, dans laquelle la surface interne dudit trou borgne (32) et la surface externe dudit corps essentiellement cylindrique (33) incluent des moyens en coopération mutuelle pour ajuster le positionnement longitudinal relatif dudit premier composant formant tête (30) par rapport audit second composant formant tête (31), en particulier un fil (40, 41) ou un mécanisme d'accrochage (38).

12. Prothèse selon la revendication 10 ou 11, dans laquelle ledit corps essentiellement cylindrique (33) inclut un trou traversant allongé (34) qui est aligné dans une direction de rotation avec un trou (35) prévu dans la surface circonférentielle dudit premier composant formant tête (30) pour recevoir une vis de fixation (36).

13. Prothèse selon l'une quelconque des revendications 1 à 12, adaptée pour l'articulation d'un radius (4) par rapport à un humérus (11),
dans laquelle ledit élément de mise en prise avec le premier os (18) est formé comme un élément de mise en prise avec un radius devant être agencé et supporté sur l'extrémité proximale d'un radius (4), et
dans laquelle ladite partie attenante (25) dudit élément de tête articulaire (16) est construite comme un évidement essentiellement concave (26) adapté pour coopérer avec l'extrémité distale de l'humérus (11).

14. Prothèse selon l'une quelconque des revendications 1 à 13, adaptée pour l'articulation d'un radius (4) par rapport à un humérus (11),
dans laquelle ledit élément de mise en prise avec le premier os (18) est formé comme un élément de mise en prise avec un radius devant être agencé et supporté sur l'extrémité proximale d'un radius (4), et
dans laquelle ladite partie attenante (25) dudit élément de tête articulaire (16) est construite comme un évidement essentiellement concave (26) adapté pour s'articuler anatomiquement avec l'extrémité distale de l'humérus (11).

15. Prothèse selon l'une quelconque des revendications 1 à 12, adaptée pour fonctionner comme une prothèse d'articulation radiocubitale,
dans laquelle ledit élément de mise en prise avec le premier os (18) est formé comme un élément de mise en prise avec un cubitus devant être agencé et supporté sur l'extrémité distale d'un cubitus (13), et
dans laquelle ladite partie attenante (25) dudit élément de tête articulaire (16) est construite comme une tête sphérique (37) adaptée pour s'articuler avec l'incision cubitale du radius (4) correspondant.
